# EUROPEAN PATENT SPECIFICATION

(11) **EP 0 419 880 B1**
(45) Date of publication and mention of the grant of the patent: **25.05.1994**
(21) Application number: 90116641.3
(22) Date of filing: 30.08.1990
(51) Int. Cl.: A61M 5/158, A61M 5/32

(54) **Medical needle device and medical apparatus having the same**
Medizinische Kanüleneinheit und damit versehene medizinische Einrichtung
Dispositif médical à canule et appareil qui le comporte

(30) Priority: 30.08.1989 JP 223725/89
(43) Date of publication of application: 03.04.1991
(73) Proprietor: TERUMO KABUSHIKI KAISHA, Tokyo 151 (JP)
(72) Inventor: Tanokura, Nobukazu, c/o Terumo Kabushiki Kaisha, Shizuoka-ken (JP)
(74) Representative: Casalonga, Axel

(56) References cited:
- US-A- 4 508 534
- US-A- 4 551 138
- US-A- 4 657 535

## Description

The present invention relates to a medical needle device of the disposable type and a medical apparatus intended to use this medical needle device.

Such a medical needle device is disclosed in U.S. Patent No. 4,657,535. It is well known as the one capable of guaranteeing its tamperproof (which means that the fact that the medical needle device was once used can be recognized later) and its sanitation. This medical needle device comprises a hub for supporting the base of a hollow needle and connecting a connection tube to the hollow needle, and a protector closed at its front end but opened at its base and serving to cover and seal the hollow needle exposed from the hub. The protector and the hub are connected to each other by a coupler provided with a thin portion which can be broken off. The coupler is connected to the protector and the hub by its blocking (which means that two members are bonded to each other by heat created at the time of heat treatment, for example heat sterilization, without using solvent, adhesive or the like). In the case where this medical needle device is to be used, the thin portion of the coupler is broken off and the protector is thus detached from the hub to expose the hollow needle outside. Therefore, the broken coupler teaches that the medical needle device was once used, thereby guaranteeing the medical needle device to be tamperproof.

When the coupler is once broken off to expose the hollow needle outside the protector in the case of this medical needle device, however, the hollow needle cannot be again housed in the protector not to easily get out of the protector. After the medical needle device is used, therefore, it is often disposed with its hollow needle exposed outside. This makes its disposing work very dangerous. Almost all of the medical needle devices which were once used are disposed without being used again particularly in these days. Therefore, the amount of these medical needle devices disposed is quite so large that the possibility of making their disposing work dangerous can be increased.

The present invention is therefore intended to eliminate the above-mentioned drawbacks.

Accordingly, the object of the present invention is to provide a medical needle device guaranteed to be tamperproof and sanitary and capable of housing a hollow needle again in a protector not to easily get out of the protector even after the medical needle device is once used. The object of the present invention is also to provide a medical apparatus intended to use the medical needle device.

The object of the present invention can be achieved by a medical needle device as defined in claim 1. Particular embodiments of the invention are specified in the dependant claims.

A medical apparatus according to the present invention comprises the above-described medical needle device, a tube communicated with the hollow needle through the hub, and a container connected to the tube to contain liquid or the like therein.

In the case of the medical needle device, the protector is separated from the hub by the opening means and the hollow needle thus exposed outside from the protector is used. After it is used, the hollow needle is again housed in the protector while the protector is connected to the hub by the connecting means not to be easily disconnected from the hub. The medical needle device can be therefore guaranteed to be tamperproof and sanitary. Even after it is used, the hollow needle can be again housed in the protector not to easily get out of the protector. This can keep workers safe while they are disposing the used medical needle devices.

By means of the feature that the pipe-like member is arranged round the coupler which is provided with a thin portion, the protector can be easily fitted onto the hub after it is separated from the hub. Further, since the pipe-like member is made of such a material that is not blocked to the coupler at the time when the medical needle device is heat-sterilized, the thin portion of the coupler can be easily broken off to easily separate the protector from the hub.

When the medical apparatus is provided with the above-described medical needle device, its disposing work can be made safe.

This invention can be more fully understood from the following detailed description when taken in conjunction with the accompanying drawings, in which:
Fig. 1 is a side view showing an example of the medical needle device according to the present invention half-sectioned along the longitudinal axis of the device;
Fig. 2 is a side view, half-sectioned, showing the center portion of the medical needle device enlarged;
Fig. 3 is a side view, half-sectioned, showing a hub with a hollow needle, and a protector separated from each other in the medical needle device shown in Fig. 1;
Fig. 4 shows a medical apparatus into which the medical needle device of the present invention is incorporated;
Fig. 5 is a side view showing the hub with the hollow needle just put back into the protector after it is used and;
Fig. 6 is a side view showing the hub with the hollow needle housed in the protector after it is used.

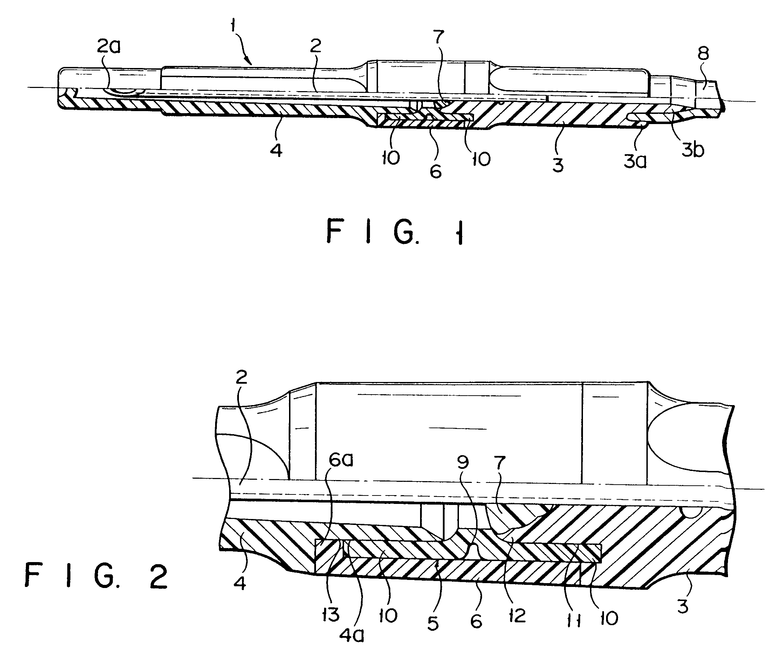

In Fig. 1, a medical needle device 1 comprises a hub 3 for supporting a hollow needle 2, a protector 4 in which the hollow needle 2 projected from the hub 3 is sealed, a breakable coupler 5 liquid-tightly arranged between the protector 4 and the hub 3, and a pipe member 6 attached to the protector 4.

A base 2b of the hollow needle 2 which has a sharp tip 2a is fixed to one end of the hub 3 by adhesive 7 such as epoxy resin. A ring-shaped recess 3a and a protrusion 3b are formed on the other end of the hub 3 and a tube 8 is connected to these ring-shaped recess 3a and protrusion 3b of the hub 3. The hollow needle 2 is thus communicated with the tube 8.

That portion of the hollow needle 2 which is projected from the hub 3 is covered and sealed by the protector 4. The coupler 5 is interposed between the hub 3 and the protector 4, as described above, to liquid-tightly connect them. They are connected to the coupler 5 by blocking at the time of heat (or autoclave) sterilization. Their connection relative to the coupler 5 is made strong by fastening action created by difference between coefficients of their thermal expansion.

As shown in Fig. 2, the coupler 5 is a pipe-like member provided with a thin portion 9 extending in the circumferential direction substantially at the center thereof. The coupler 5 is formed separately from the hub 3 and the protector 4. This enables the portion 9 of the coupler 5 to be made accurately thin enough to be easily twisted and broken. One of the pipe-like leg portions 10 extending from the thin portion 9 of the coupler 5 to both sides thereof is fitted into a recess 11 on the front end of the hub 3 while the other into the base 4a of the protector 4. The material which form the leg portions 10 of the coupler 5 is then shrunk by heating process in the course of the heat sterilization to fasten a protrusion 12 of the hub 3 and the base 4a of the protector 4 while adhering to them by its blocking. This guarantees the medical needle device to be liquid-tight.

When the liquid-tightness and tamperproof of the medical needle device are not sufficient only by the fastening and blocking of the coupler 5 relative to the hub 3 and the protector 4, adhesive can be applied between the coupler 5 and the hub 3 and between the coupler 5 and the protector 4. It is preferable in this case to use adhesive of the non-solvent type, particularly more preferable to use adhesive of the UV curing type.

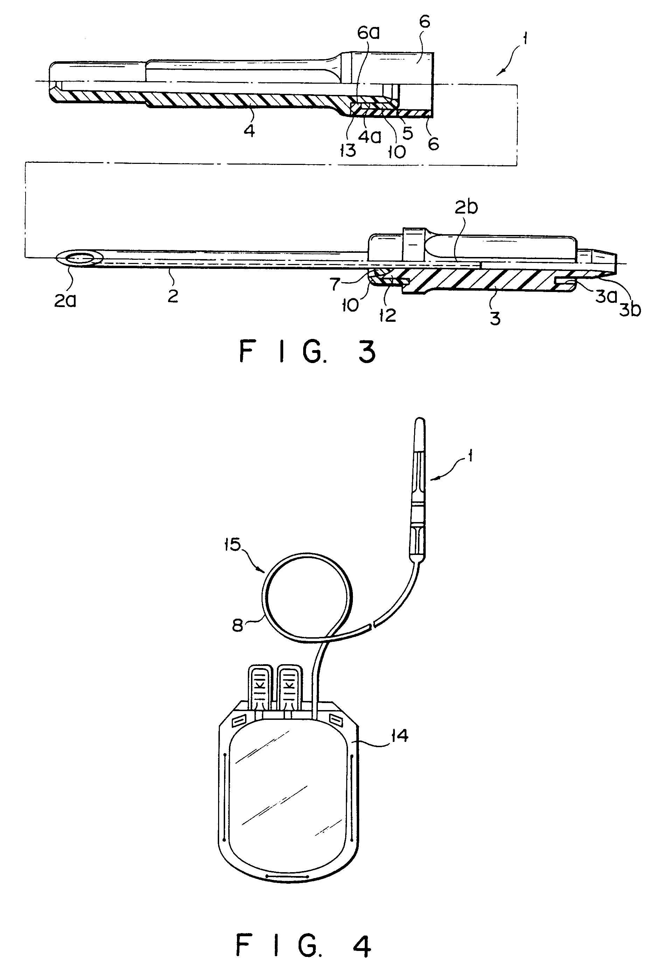

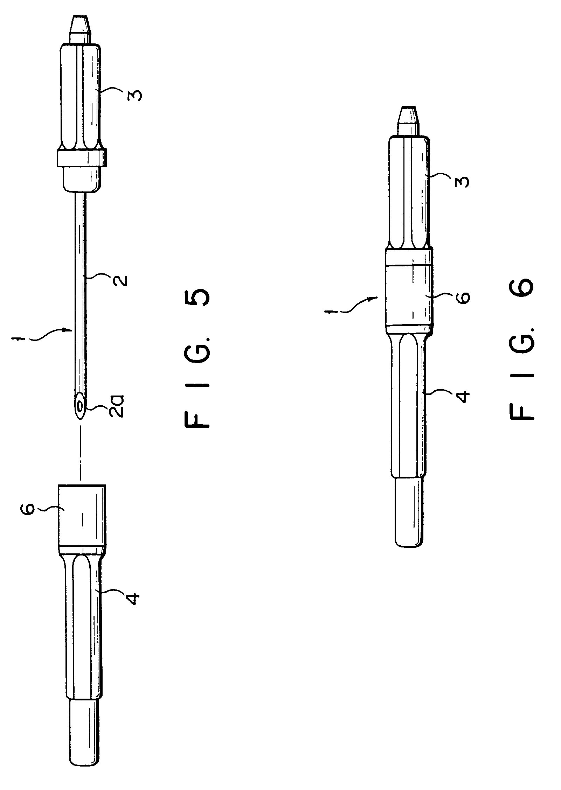

The pipe-like member 6 is fitted onto the coupler 5 to cover substantially the whole of the latter. A flange 6a of the pipe-like member 6 is embedded in a groove 13 between the front end 4a of the protector 4 and the coupler 5 and the pipe-like member 6 is thus fixed to the protector 4. When the thin portion 9 of the coupler 5 is broken to separate the hub 3 provided with the hollow needle 2 from the protector 4 as shown in Fig. 3, therefore, the pipe-like member 6 belongs to the protector 4. After the medical needle device 1 is used, the hollow-needle-attached hub 3 is put back into the pipe-like-member-attached protector 4 and then disposed as it is (see Fig. 6).

The coupler 5, the hub 3 and the protector 4 are connected to one another by their blocking, as described above. Materials of which these components are made are therefore of the type capable of blocking to one another. However, the pipe-like member 6 is attached to and detached from a part of the coupler 5 which is left on the side of the hub 3, as described above. Material of which the pipe-like member 6 is made is of the type capable of not blocking to the coupler 5.

It is preferable that the materials of which the coupler 5, the hub 3 and the protector 4 are made are different in heat shrinking rate and that their blocking is made strong by the difference of their heat shrinking rates. The material of which the hub 3 and the protector 4 are made is of the type not shrunk by heat. Polycarbonate, resins of the vinyl chloride group and MBS resins can be mentioned to use as the material and it is preferable to select polycarbonate because it is not deformed at the time of the heat sterilization and because it has high heat resistance. The coupler 5 is fitted into the recess 11 of the hub 3 and contacted with the protrusion 12 of the hub 3 and the base 4a of the protector 4. It is therefore needed that the coupler 5 is connected to these components by blocking with its heat shrinkage to guarantee the medical needle device 1 to be liquid-tight and tamperproof. Resins of the vinyl chloride group, polycarbonate and MBS resins can be mentioned as being suitable for use as the material of which the coupler 5 is made. Polyvinyl chloride is more suitable because it can be easily twisted and broken.

The pipe-like member 6 is made of such material that is neither shrunk by heat nor blocked relative to the matter or polyvinyl chloride of which the coupler 5 is made.

The tube 8 is made of resin of the vinyl chloride group and its adhesion relative to the hub 3 which is made of polycarbonate, for example, is carried out in such a way that it is fitted into the ring-shaped recess 3a of the hub 3 and that it is blocked to the protrusion 3b of the hub 3 located inside it by heat created at the time of the heat sterilization.

The protector 4 is asked to be transparent because the hollow needle 2 sealed in the protector 4 must be recognized. Polycarbonate is therefore more preferable than polyvinyl chloride. Polycarbonate is difficultly deformed by heat and this makes it more suitable for being used as the material of which the protector 4, usually thin, is made. When polyvinyl chloride is used, it is easily deformed by heat to thereby create the possibility of its damaging the hollow needle 2.

The medical needle device 1 having the above-described arrangement is connected to a blood bag 14 through the tube 8 and used as a medical apparatus 15, as shown in Fig. 4. When the medical apparatus 15 is to be used, the hub 3 and the protector 4 are twisted in reverse directions by fingers to break off the thin ring-shaped portion 9 of the coupler 5. A part of the coupler 5, the protector 4 and the pipe-like member 6 are thus broken off from the hub 3 and that part of the coupler 5 which is left on the hub 3 to thereby expose the hollow needle 2 outside, as shown in Fig. 5. The tip 2a of the hollow needle 2 is stuck into a patient to collect blood from him according to the usual manner. The blood is collected into the blood bag 14 through the hollow needle 2 and the connection tube 8. After the blood thus collected in the blood bag 14 is used up later and the medical apparatus 15 therefore becomes unnecessary, the hollow needle 2 is housed in the protector 4 and the pipe-like member 6 is fitted onto the hub 3 (see Fig. 6). The medical needle device 1 is thus disposed together with the blood bag 14 and the tube 8.

## Claims

1. A medical needle device comprising a hollow needle (2), a hub (3) for supporting the base of the hollow needle (2), a protector (4) for covering and sealing the hollow needle (2) projecting from the hub (3), a coupler (5) for liquid-tightly connecting the protector (4) with the hub (3) by blocking, the coupler comprising a breakable thin portion (9) for separating the hub (3) from the protector (4) when broken off, and a connecting means (6) for connecting the hub (3) and the protector (4), the connecting means (6) being arranged around the coupler (5) and being made of a material that does not adhere to the coupler (5) by blocking,
**characterised** in that
- the connecting means is a pipe-like member (6) separately formed from the hub (3) and the protector (4),
- a recess (13) is formed between an end of the coupler (5) and an opposite portion of one of the hub (3) and the protector (4), and
- an inwardly directed flange projection (6a) is formed at an end of the pipe-like member (6) and is engaged with the recess (13) so as to fix the pipe-like member (6) to one of the hub (3) and the protector (4) such that it is left on the hub (3) or the protector (4) after the thin portion is broken off.

2. The medical needle device according to claim 1,
**characterised** in that the pipe-like member (6) is fixed to the protector (4).

3. The medical needle device according to claim 1 or 2,
**characterised** in that the coupler (5) is made of one or more materials selected from the group consisting of vinyl chloride resins, polycarbonate and MBS resin.

4. The medical needle device according to claim 3,
**characterised** in that the coupler (5) is made of polyvinyl chloride.

5. A medical apparatus comprising:
- medical needle device according to any of the preceeding claims,
- a tube (8) communicated with the hollow needle (2) through the hub (3); and
- a container (14) connected to the tube (8) to contain a fluid therein.

## Patentansprüche

1. Medizinische Kanüleneinheit, umfassend eine Kanüle (2), eine Abdeckkappe (3) zum Haltern der Basis der Kanüle (2), eine von der Abdeckkappe (3) vorstehende Schutzvorrichtung (4) zum Abdecken und Abdichten der Kanüle (2), ein Verbindungsmittel (5) zum flüssigkeitsdichten Verbinden der Schutzvorrichtung (4) mit der Abdeckkappe (3) durch Blockieren, wobei das Verbindungsmittel einen zerbrechbaren dünnen Abschnitt (9) zum Trennen der Abdeckkappe (3) von der Schutzvorrichtung (4) nach dem Abbrechen und eine Verbindungseinrichtung (6) zum Verbinden der Abdeckkappe (3) und der Schutzvorrichtung (4) umfaßt, wobei die Verbindungseinrichtung (6) um das Verbindungsmittel (5) angeordnet ist und aus einem Material hergestellt ist, das am Verbindungsmittel (5) nicht durch Blockieren hängenbleibt,
dadurch **gekennzeichnet**, daß
- die Verbindungseinrichtung ein rohrartiges Element (6) ist, das von der Abdeckkappe (3) und der Schutzvorrichtung (4) getrennt gebildet ist,
- zwischen einem Ende des Verbindungsmittels (5) und einem entgegengesetzten Abschnitt der Abdeckkappe (3) oder der Schutzvorrichtung (4) eine Aussparung (13) gebildet ist und
- an einem Ende des rohrartigen Elementes (6) ein nach innen gerichteter Flanschvorsprung (6a) gebildet ist und sich mit der Aussparung (13) in Eingriff befindet, um das rohrartige Element (6) an der Abdeckkappe (3) oder an der Schutzvorrichtung (4) zu befestigen derart, daß es an der Abdeckkappe (3) oder der Schutzvorrichtung (4) bleibt, nachdem der dünne Abschnitt abgebrochen ist.

2. Medizinische Kanüleneinheit nach Anspruch 1, dadurch **gekennzeichnet,** daß das rohrartige Element (6) an der Schutzvorrichtung (4) befestigt ist.

3. Medizinische Kanüleneinheit nach Anspruch 1 oder 2, dadurch **gekennzeichnet,** daß das Verbindungsmittel (5) aus einem oder mehreren Materialien hergestellt ist, die aus der aus Vinylchloridharzen, Polycarbonat und MBS-Harz bestehenden Gruppe ausgewählt sind.

4. Medizinische Kanüleneinheit nach Anspruch 3, dadurch **gekennzeichnet,** daß das Verbindungsmittel (5) aus Polyvinylchlorid hergestellt ist.

5. Medizinische Vorrichtung, umfassend
- eine medizinische Kanüleneinheit nach einem beliebigen der vorhergehenden Ansprüche,
- einen Schlauch (8), der mit der Kanüle (2) durch die Abdeckkappe (3) in Verbindung steht; und
- einen Behälter (14), der mit dem Schlauch (8) verbunden ist, um darin eine Flüssigkeit zu enthalten.

## Revendications

1. Dispositif medical à aiguille comprenant une aiguille creuse (2), un manchon (3) pour supporter la base de l'aiguille creuse (2), un élément de protection (4) pour recouvrir et enfermer de façon étanche l'aiguille creuse (2) faisant saillie du manchon (3), un élément d'accouplement (5) pour raccorder de façon étanche aux liquides l'élément de protection (4) au manchon (3) par blocage, ledit élément d'accouplement comprenant une partie mince cassable (9) pour séparer le manchon (3) de l'élément de protection (4) lorsqu'on la rompt, et un moyen de raccorderment (6) pour raccorder le manchon (3) et l'élément de protection(4), ledit moyen de raccordement (6) étant disposé autour de l'élément d'accouplement (5) et étant formé d'un matériau qui n'adhère pas à l'élément d'accouplement (5) par blocage, caractérisé en ce que
- le moyen de raccordement est un élément (6) analogue à un tube formé séparément du manchon (3) et de l'élément de protection (4),
- un évidement (13) est formé de façon adjacente à une extrémité de l'élément d'accouplement (5) dans une partie du manchon (3) ou de l'élément de protection (4), et en ce que
- un rebord (6a) faisant saillie vers l'intérieur est formé à une extrémité de l'élément (6) analogue à un tube et est engagé dans ledit évidement (13) de manière à fixer l'élément (6) analogue à un tube au manchon (3) ou à l'élément de protection (4), de telle sorte qu'il reste sur le manchon (3) ou sur l'élément de protection après que ladite portion mince a été rompue.

2. Dispositif médical à aiguille selon la revendication 1, caractérisé en ce que l'élément (6) analogue à un tube est fixé à l'élément de protection (4).

3. Dispositif médical à aiguille selon la revendication 1 ou 2, caractérisé en ce que l'élément d'accouplement (5) est formé d'un ou plusieurs matériaux choisis parmi le groupe comprenant les résines de chlorure de vinyle, un polycarbonate et une résine MBS.

4. Dispositif médical à aiguille selon la revendication 3, caractérisé en ce que l'élément d'accouplement (5) est formé de poly(chlorure de vinyle).

5. Appareil médical comprenant :
- un dispositif médical à aiguille selon l'une quelconque des revendications précédentes,
- un tube (8) communiquant avec l'aiguille creuse (2) à travers le manchon (3); et
- un récipeient (14) raccordé au tube (8) pour contenir un fluide.
